# EUROPEAN PATENT APPLICATION

(11) **EP 4 454 656 A1**
(43) Date of publication of application: **30.10.2024**
(21) Application number: 21968806.6
(22) Date of filing: 20.12.2021
(51) Int. Cl.: A61K 36/73, A61K 31/706

(54) **LIQUID COMPOSITION AND COMPOSITION FOR ACTIVATING SIRTUIN**

(71) Applicant: Dream Power Incorporated, Osaka-shi, Osaka 541-0048 (JP)
(72) Inventor: IKOMA, Yuuki, Osaka-shi, Osaka 541-0048 (JP); YAMANE, Takuya, Osaka-shi, Osaka 530-0005 (JP)
(74) Representative: Regimbeau
(86) International application number: PCT/JP2021/047040
(87) International publication number: WO 2023/119368

(57) **Abstract**

Provided is a novel composition that contains nicotinamide mononucleotide and has excellent biofunctional activity. The present invention relates to a liquid composition that contains nicotinamide mononucleotide and an aronia extract.

## Description

### TECHNICAL FIELD

The present invention relates to a liquid composition and a composition for activating sirtuin, containing nicotinamide mononucleotide.

### BACKGROUND ART

Nicotinamide mononucleotide is a biosynthetic intermediate metabolite of coenzyme NAD+. In Non Patent Literature 1 (Mihoko Yoshino, Jun Yoshino, Brandon D Kayser, Gary J Patti, Michael P Franczyk, Kathryn F Mills, Miriam Sindelar, Terri Pietka, Bruce W Patterson, Shin-Ichiro Imai, Samuel Klein, Nicotinamide mononucleotide increases muscle insulin sensitivity in prediabetic women, Science 2021, 372, 1224-1229), in human clinical trials for oral intake, nicotinamide mononucleotide has been reported to have the actions of improving obesity, preventing diabetes, and improving muscle insulin sensitivity. Also, in Non Patent Literature 2 (Kathryn F. Mills, Shohei Yoshida, Liana R. Stein, Koji Uchida, Jun Yoshino, Shin-ichiro Imai, Long-Term Administration of Nicotinamide Mononucleotide Mitigates Age-Associated Physiological Decline in Mice, Cell Metabolism 2016, 24, 795-806), in a test with mice for oral intake, it has been reported that nicotinamide mononucleotide has the actions of suppressing weight gain, improving insulin sensitivity, improving lipid metabolism, improving visual ability, increasing bone density, and increasing immune function. In Non Patent Literature 3 (Jun Yoshino, Kathryn F. Mills, Myeong Jin Yoon, Shin-ichiro Imai, Nicotinamide Mononucleotide, a Key NAD+ Intermediate, Treats the Pathophysiology of Diet- and Age-Induced Diabetes in Mice, Cell Metabolism 2011, 14, 528-536), in a test with mice, it has been reported that nicotinamide mononucleotide has a sirtuin-activating action.

### CITATION LIST

### NON PATENT LITERATURE

NPL 1: Mihoko Yoshino, Jun Yoshino, Brandon D Kayser, Gary J Patti, Michael P Franczyk, Kathryn F Mills, Miriam Sindelar, Terri Pietka, Bruce W Patterson, Shin-Ichiro Imai, Samuel Klein, Nicotinamide mononucleotide increases muscle insulin sensitivity in prediabetic women, Science 2021, 372, 1224-1229
NPL 2: Kathryn F. Mills, Shohei Yoshida, Liana R. Stein, Koji Uchida, Jun Yoshino, Shin-ichiro Imai, Long-Term Administration of Nicotinamide Mononucleotide Mitigates Age-Associated Physiological Decline in Mice, Cell Metabolism 2016, 24, 795-806
NPL 3: Jun Yoshino, Kathryn F. Mills, Myeong Jin Yoon, Shin-ichiro Imai, Nicotinamide Mononucleotide, a Key NAD+ Intermediate, Treats the Pathophysiology of Diet- and Age-Induced Diabetes in Mice, Cell Metabolism 2011, 14, 528-536

### SUMMARY OF INVENTION

### TECHNICAL PROBLEM

An object of the present invention is to provide a novel composition that contains nicotinamide mononucleotide and has excellent biofunctional activity.

### SOLUTION TO PROBLEM

[1] A liquid composition containing nicotinamide mononucleotide and an aronia extract.
[2] The liquid composition according to [1], which is for suppressing aging.
[3] The liquid composition according to [2], which has a sirtuin-activating action.
[4] A composition for activating sirtuin, containing nicotinamide mononucleotide and an aronia extract.
[5] The composition according to [4], wherein the sirtuin is sirtuin 1.
[6] The composition according to any one of [1] to [5], which is for oral intake.

### ADVANTAGEOUS EFFECTS OF INVENTION

The present invention provides a novel composition that contains nicotinamide mononucleotide and has excellent biofunctional activity.

### BRIEF DESCRIPTION OF DRAWINGS

Fig. 1 is a diagram showing the result of fluorescence intensity measured at predetermined time intervals.
Fig. 2 is a diagram showing relative values of SIRT1 enzyme activity.
Fig. 3 is a diagram showing the calculated value of SIRT1 enzyme activity by adding the results of a liquid sample 2 and a liquid sample 3, and the measured value of a liquid sample 1.

### DESCRIPTION OF EMBODIMENTS

### [Composition]

The composition of the present invention contains nicotinamide nucleotide (hereinafter also referred to as "NMN") and an aronia extract. The composition of the present invention is preferably a composition for oral intake.

### <NMN>

NMN (chemical formula: C₁₁H₁₅N₂O₈P) has two optical isomers, α and β, and β-NMN (CAS number: 1094-61-7) is used in the present invention. The structure of β-NMN is shown below.

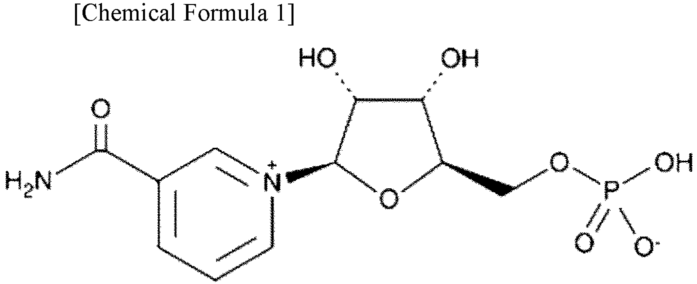

β-NMN may be prepared by any method. For example, artificially synthesized β-NMN purified by a chemical synthesis method, an enzymatic method, a fermentation method, or the like can be used. In addition, β-NMN is a component widely present in living organisms, and thus β-NMN obtained by extraction and purification from natural raw materials such as animals, plants, and microorganisms can also be used. In addition, commercially available purified β-NMN may be used.

As a chemical synthesis method for synthesizing β-NMN, for example, β-NMN can be produced by reacting nicotinamide with L-ribose tetraacetate and phosphorylating the obtained nicotinamide mononucleoside. In addition, as an enzymatic method, for example, β-NMN can be produced from nicotinamide and 5'-phosphoribosyl-1'-pyrophosphate (PRPP) with nicotinamide phosphoribosyltransferase (NAMPT). As a fermentation method, for example, β-NMN can be produced from nicotinamide with the metabolic system of a microorganism expressing NAMPT.

NMN used in the composition of the present invention may be a pharmacologically acceptable salt of β-NMN. The pharmacologically acceptable salt of β-NMN may be an inorganic acid salt or an organic acid salt having a basic site such as an amine. Examples of the acid constituting such an acid salt include acetic acid, benzenesulfonic acid, benzoic acid, camphorsulfonic acid, citric acid, ethenesulfonic acid, fumaric acid, gluconic acid, glutamic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phosphoric acid, succinic acid, sulfuric acid, tartaric acid, and p-toluenesulfonic acid. In addition, the pharmacologically acceptable salt of β-NMN may be an alkali salt or an organic salt having an acidic site such as a carboxylic acid. Examples of the base constituting such an acid salt include an alkali metal salt or an alkaline earth metal salt, and examples thereof include a derivative from a base such as sodium hydride, potassium hydroxide, calcium hydroxide, aluminum hydroxide, lithium hydroxide, magnesium hydroxide, zinc hydroxide, ammonia, trimethylammonia, triethylammonia, ethylenediamine, lysine, arginine, ornithine, choline, N,N'-dibenzylethylenediamine, chloroprocaine, procaine, diethanolamine, N-benzylphenethylamine, diethylamine, piperazine, tris(hydroxymethyl)-aminomethane, and tetramethylammonium hydroxide.

The NMN used in the composition of the present invention may be free β-NMN or a solvate of a pharmacologically acceptable salt of β-NMN. Examples of the solvent to form the solvate include water and ethanol.

### <Aronia extract>

Aronia is a plant of the genus Aronia in the family Rosaceae, and is preferably selected from one or more of Aronia albutifolia, Aronia melanocarpa, and Aronia prunifolia. The extract herein includes a compressed product obtained by pressing a plant as it is, a plant extract itself, and a product derived from a plant extract. As the plant extract, any of a press effluent, a steam distillate, a distillate, and a solvent extract can be used. The product derived from a plant extract includes a fraction obtained by fractionating and purifying a plant extract, a product obtained by removing a solvent of a purified product, and the like.

The solvent used for solvent extraction is preferably a polar solvent, and preferable examples thereof include water, alcohols such as ethanol and isopropanol, esters such as ethyl acetate, and ketones such as acetone and methyl ethyl ketone, and among these, an aqueous solution containing ethanol is preferable. The content in this case is preferably 50% by mass to 95% by mass, more preferably 60% by mass to 90% by mass, based on the total amount of the solvent. Extraction may be performed by adding 1 to 10 parts by mass of a solvent to 1 part by mass of the plant or its dried product, and immersing for several days at room temperature or for several hours at a temperature near the boiling point. Insoluble matter may be removed by filtration if necessary. Concentrating may be performed by distillation under reduced pressure.

There are no particular limitations on the plant parts used to produce the aronia extract, and the whole plant can be used, but of course, it is also possible to use only parts such as the plant bodies, aerial parts, rhizomes, tree trunks, leaves, stems, flower spikes, flower buds, and fruits. Preferable examples thereof include fruits. As the aronia extract, it is particularly preferable to use fruit juice obtained by directly pressing the fruit part, from the viewpoint of effectively utilizing the active ingredients.

The aronia extract can be prepared and used as described above, or commercially available one can be purchased and used.

### <Food composition, pharmaceutical composition, cosmetic composition>

The composition of the present invention is preferably a food composition, a pharmaceutical composition, or a cosmetic composition.

Examples of the food compositions include foods (including foods with nutritional function claims, foods for specified health uses, and the like), nutritional supplements, nutrients, drinks, and feeds. Examples of the pharmaceutical compositions include veterinary drugs, quasi-drugs, pharmaceuticals, therapeutic drugs, and preventive drugs.

Examples of the foods include: dairy products such as cheese, modified milk powder, ice cream, and yogurt; confectionery such as chocolate, cookies, biscuits, candies, Japanese sweets, rice crackers, cakes, pies, and pudding; flour products such as bread and noodles; rice products such as rice porridge and cooked rice; and seasonings such as soy sauce, miso, mayonnaise, and dressing. The foods may be processed marine products, processed agricultural products, or processed livestock products.

Examples of the beverages include tea, coffee, milk, milk drinks, fruit juice drinks, juices, lactic acid drinks, soft drinks, nutritional drinks, and beauty drinks.

These compositions can contain various additives depending on the type thereof. Examples of the additives include sweeteners. Examples of the sweeteners include glucose, fructose, sucrose, maltose, sorbitol, stevioside, rubusoside, corn syrup, lactose, and maltitol.

Examples of other additives include citric acid, tartaric acid, malic acid, succinic acid, lactic acid, L-ascorbic acid, dl-α-tocopherol, sodium erythorbate, glycerin, propylene glycol, glycerin fatty acid ester, magnesium stearate, calcium stearate, cellulose derivatives such as hydroxypropyl cellulose, crystalline cellulose, silicon dioxide, polyglycerin fatty acid ester, sucrose fatty acid ester, sorbitan fatty acid ester, gum arabic, carrageenan, casein, gelatin, pectin, agar, vitamin B, nicotinamide, calcium pantothenate, amino acids, calcium salts, dyes, fragrances, and preservatives.

Examples of nutritional supplements, nutrients, or pharmaceutical compositions include tablet preparations, capsule preparations, granule preparations, powder preparations, fine granule preparations, chewable tablets, pill preparations, lozenge preparations, sublingual tablets, liquid preparations, ampule preparations, gum preparations, drop preparations, ointments, cream preparations, emulsion preparations, suspension preparations, jelly preparations, syrup, and liquid preparations.

For preparation, additives such as excipients, binders, disintegrants, lubricants, stabilizers, flavoring agents, diluents, and injection solvents can be used.

Examples of the excipients include: cellulose derivatives such as crystalline cellulose, hydroxypropylcellulose, hydroxypropylmethylcellulose, carboxymethylcellulose, and carboxymethylcellulose calcium; gum arabic; dextran; pullulan; silicate derivatives such as light anhydrous silicic acid, synthetic aluminum silicate, and magnesium metasilicate aluminate; phosphate derivatives such as calcium phosphate; carbonate derivatives such as calcium carbonate; and sulfate derivatives such as calcium sulfate.

Examples of the binder include, gelatin, polyvinylpyrrolidone, and magrogol, in addition to the above-described excipients.

Examples of the disintegrants include chemically modified starch or cellulose derivatives such as croscarmellose sodium, sodium carboxymethyl starch, and crosslinked polyvinylpyrrolidone, in addition to the above-described excipients.

Examples of the lubricants include: talc; stearic acid; stearic acid metal salts such as calcium stearate and magnesium stearate; colloidal silica; waxes such as begum and gaylow; boric acid; glycol; carboxylic acids such as fumaric acid and adipic acid; carboxylic acid sodium salts such as sodium benzoate; sulfuric acid salts such as sodium sulfate; leucine; lauryl sulfates such as sodium lauryl sulfate and magnesium lauryl sulfate; silicates such as silicic anhydride and silicic acid hydrate; and starch derivatives.

Examples of the stabilizers include: paraoxybenzoic acid esters such as methylparaben and propylparaben; alcohols such as chlorobutanol, benzyl alcohol, and phenylethyl alcohol; benzalkonium chloride; acetic anhydride; and sorbic acid.

Examples of the flavoring agents include sweeteners, acidulants, and fragrances. Other components, drugs, or the like may be contained in the preparation in order to reinforce or enhance the usefulness of the present invention.

When the composition is a food composition such as a nutritional supplement or nutrient, or a pharmaceutical composition, the total content of NMN and the aronia extract in the composition is preferably 0.1% by mass to 100% by mass, more preferably 10% by mass to 90% by mass, still more preferably 40% by mass to 80% by mass.

Cosmetic composition refers to a composition that is applied to the body by rubbing, spraying, or the like, in order to keep the skin or hair healthy, or to cleanse, beautify, increase attractiveness, or change the appearance of the body. Examples of the cosmetic compositions include basic cosmetics, makeup cosmetics, and hair cosmetics. In the present description, "cosmetics" includes those classified as quasi-drugs, such as medicinal cosmetics, as defined in the Act on Ensuring the Quality, Efficacy, and Safety of Products Including Pharmaceuticals and Medical Devices.

### (Liquid composition)

Examples of one embodiment of the composition of the present invention include a liquid composition. In addition, examples of one embodiment of the liquid composition of the present invention includes a beverage. It is known that NMN is easily decomposed in a humid environment. According to the present invention, the decomposition of NMN can be suppressed by using together with the aronia extract even in a beverage with a high water content.

In addition to NMN and an aronia extract, to the beverage, various ingredients that are normally blended to beverages, such as: sweeteners; acidulants; vitamins such as vitamin A, vitamin B, and vitamin E; antioxidants; preservatives such as sodium benzoate; thickeners; emulsifiers; minerals such as sodium, calcium, magnesium, potassium, zinc, iron, manganese, and copper; amino acids such as monosodium glutamate, lysine hydrochloride, carnitine citrulline, arginine, and ornithine and the salts thereof; fragrances such as herbal fragrances; fruit juices such as apple, grape, grapefruit, pineapple, peach, lemon, plum, and orange and the concentrated juices thereof; royal jelly; guarana extract; caffeine; garlic extract; maca extract; herbal medicine; and seaweed extract can be added as desired within the limits that do not impair the effects of the present invention.

Examples of sweeteners include sugars such as fructose, sucrose, glucose, granulated sugar, lactose, and maltose, and low-intensity sweeteners such as xylitol or D-sorbitol. Examples of the acidulant include citric acid salts such as trisodium citrate, anhydrous citric acid, adipic acid, gluconic acid, succinic acid, tartaric acid, fumaric acid, malic acid, phytic acid, ascorbic acid, or the salts thereof.

The beverage can be obtained, for example, by uniformly mixing NMN and aronia extracts, and other ingredients added as necessary, in water according to a conventional method. The order in which each ingredient is added is not limited, and for example, the beverage can be obtained by adding other ingredients to a beverage containing an aronia extract. The adjusted beverage may be heat sterilized and packed into a container as a packaged beverage. The total amount of the beverage in the packaged beverage is not limited, but is, for example, 5 mL or more and 500 mL or less, preferably 10 mL or more and 100 mL or less. The timing of blending each ingredient is not limited to before the sterilization process, and specific ingredients such as easily decomposed NMN or aromatic ingredients with relatively high volatility may be blended after the sterilization process.

The storage temperature of the beverage is not particularly limited, but can be, for example, 1°C or more and 40°C or less, preferably 2°C or more and 10°C or less. The beverage may be stored frozen. The beverage of the present invention exhibits the effect of suppressing the decomposition of NMN even when stored at room temperature of 25°C or more.

Examples of the container include sealed containers made of single materials such as glass, paper, plastic (polyethylene terephthalate and the like), aluminum, and steel, or composite materials or laminated materials thereof. In addition, the type of the container is not particularly limited, and examples thereof include a plastic bottle, an aluminum can, a steel can, a paper pack, a chilled cup, and a bottle. From the viewpoints such as handling, distribution, and stability, the container is preferably a paper pack or a bottle.

The total amount of NMN contained in the beverage is preferably 0.001 mg/mL or more, more preferably 0.01 mg/mL or more based on the total amount of the beverage. The content of NMN may be, for example, 1.0 mg/mL or less, or 0.1 mg/mL or less. The present invention can suppress the decomposition of NMN in the beverage, thus allowing decomposition of NMN in the beverage to be suppressed during storage and transportation, and NMN to be efficiently administered to the recipient.

The total amount of the aronia extract contained in the beverage is preferably 1.0% by mass or more, more preferably 5.0% by mass or more, and still more preferably 50% by mass or more, based on the total amount of the beverage excluding solid additives such as NMN contained in the beverage. The upper limit is, for example, 100% by mass or less, or 99% by mass or less.

The beverage may be intaken per meal, may be intaken one time, or may be intaken multiple times.

### (Feed)

Examples of the feed include pet food, livestock feed, and fish feed. The feed composition can be produced by mixing lactic acid compounds and fructose with general feeds or the raw materials thereof, such as grains, meal, bran, fish meal, bone meal, fats and oils, skim milk powder, whey, mineral feed, and yeast. The produced feed can be orally administered to general mammals, livestock, farmed fish, pets, and the like.

### (Target for administration)

The composition of the present invention can be administered to humans and non-human animals. Examples of non-human animals include mammals such as cows, pigs, horses, sheep, goats, donkeys, monkeys, dogs, cats, rabbits, mice, rats, hamsters, and guinea pigs. The oral composition of the present invention is preferably one that is administered and intaken by humans, livestock, laboratory animals, and pets, and more preferably one that is administered and intaken by humans.

### <Application>

The composition of the present invention can be used for sirtuin activation. In the present description, "sirtuin" refers to sirtuin proteins and homologs thereof.

In the present description, "sirtuin activation" includes, for example, enhancing sirtuin expression. "Sirtuin expression" refers to both expression at the sirtuin gene transcript level and expression at the sirtuin protein level.

In the present description, "sirtuin gene" means a gene encoding sirtuin.

In the present description, "enhanced sirtuin expression" refers to an increase in sirtuin gene transcription products and/or sirtuin proteins, such as activation of gene transcription and/or translation, improvement in the stability of transcription products and/or proteins, or inhibition of decomposition of transcription products and/or proteins.

The degree of sirtuin activation can be determined from the fact that for example, activation of transcription and/or translation of sirtuin genes (for example, activation of sirtuin enzymes) is increased, for example, with a statistically significant difference (for example, Student's t-test), compared to a control, or increased by a certain percentage (for example, tens of percent, hundreds of percent) or more.

The composition of the present invention has an excellent effect on sirtuin activation by containing NMN and the aronia extract, as compared with the degree of sirtuin activation of the composition containing only one of them, or as compared with the degree of sirtuin activation by adding both of them, which is predicted by adding the degree of sirtuin activation of the composition containing only one of them with the other degree.

The animal from which the sirtuin is derived is not particularly limited, and may be an animal other than human, but is preferably human. In humans, sirtuins 1 to 7 (SIRT1 to SIRT7) are known. In the present invention, the sirtuin is preferably sirtuin 1 (SIRT1).

Sirtuins are known to be involved in suppressing cellular aging and extending lifespan due to calorie restriction, and SIRT1, the most researched sirtuin, is known to be involved in a wide variety of biological phenomena, including inhibition of cellular aging, stress resistance, metabolic regulation, anti-inflammation, cancer and cell differentiation, cell migration, depression, neurodegenerative diseases, and diabetes. For example, it is known that inhibiting SIRT1 induces cellular aging in vascular endothelial cells, and overexpressing can avoid oxidative stress-induced cellular aging (SIRT1 modulates premature senescence-like phenotype in human endothelial cells., J. Mol. Cell Cardiol., 43, 571-579 (2007)).

The composition of the present invention has a sirtuin-activating action, particularly a SIRT1-activating action, and thus can be used for activating sirtuin, and can be suitably used for SIRT1 activation. In addition, the composition of the present invention has a sirtuin-activating action, and particularly has a SIRT1-activating action, and thus can be used for suppressing aging. As an aging-suppressing action, the composition can be used to prevent cell death, suppress cellular aging, extend cell lifespan, prevent cancer cell development, and the like.

The composition of the present invention can also be used for, as an application in combination of the above application or as an application different from the above application, at least one application selected from the group consisting of, for example, improving obesity, preventing diabetes, suppressing weight gain, improving insulin sensitivity, improving lipid metabolism, improving visual ability, increasing bone density, and increasing immune function (refer to Non Patent Literatures 1 and 2).

The composition of the present invention may be labeled with descriptions regarding its application, efficacy, effect, function, type of active ingredient, type of functional ingredient, intake method, and the like. "Label" means the appropriate label for each of pharmaceutical compositions, health foods (dietary supplements, foods with nutritional function claims, foods for the sick, foods for specified health uses, products with functional claims, and the like), supplements, food for the sick (hospital food, sick food, nursing care food, and the like). In addition, "label" includes all labels for informing consumers of the application, efficacy, effect, function, and the like of the composition of the present invention. This label may be any label that makes it possible to recall or analogize the action of the composition of the present invention described above, and may include all labels regardless of the purpose of the label, the content of the label, the object or medium to be labelled, and the like. Examples thereof include labelling on product packaging/containers, labelling on product-related advertisements, price lists, or transaction documents, displaying or distributing these, or providing these by electromagnetic (internet, or the like) methods.

When the composition of the present invention is an oral composition, the amount of administration and intake is appropriately selected and determined depending on the species, age, body weight, symptoms, degree of disease, administration schedule, preparation form, and the like of the animal to be administered. For example, the daily dose per adult can be administered as the amount of β-NMN once or in divided doses, so as to be 0.1 mg to 50 g, preferably 0.5 mg to 35 g, more preferably 10 mg to 25 g, still more preferably 100 mg to 10 g.

β-NMN is a biological component and is also included in food, and thus is highly safe and suitable for long-term continuous intake.

### Example

Hereinafter, the present invention will be explained in more detail with reference to test examples, but the present invention is not limited thereto.

### (Liquid sample 1)

A liquid sample 1 was prepared by dissolving 0.5 µg of β-NMN in 5 µL of a fruit juice beverage made of an aronia extract (manufactured by Hokusan Co. Ltd., 100% aronia fruit juice).

### (Liquid sample 2)

A liquid sample 2 was prepared by dissolving 0.5 µg of β-NMN in 5 µL of water.

### (Liquid sample 3)

5 µL of a fruit juice beverage made of an aronia extract (manufactured by Hokusan Co. Ltd., 100% aronia fruit juice) was prepared as a liquid sample 3.

### [Test 1]

In test 1, the SIRT1-activating action was evaluated by measuring SIRT1 mRNA in human neuroblasts, as shown below. The above liquid samples 1 to 3 were used as samples.

Human neuroblasts (SH-SY5Y) were seeded in a 10 cm dish at 4.0 × 10⁵ cells per well. Dulbecco's modified Eagle/Ham's F-12 (DMEM/Ham's F-12) medium supplemented with 10% fetal bovine serum (FBS) was used as the seeding medium. After 24 hours, liquid samples 1 to 3 were added and a control with no liquid sample added was also prepared, incubation was performed at a temperature of 37°C and 5% CO₂, then the medium was discarded, washing was performed with PBS, LysisBuffer was added, and the cells were collected into a Falcon tube with a cell scraper. After stirring with a vortex and keeping on ice, a sucrose solution was added and centrifugation was performed. The supernatant was discarded, 10 mM Tris-HCl (pH 7.5) and 10 mM NaCl were added to suspend, and the mixture was transferred to a microtube and centrifuged. The supernatant was discarded and kept on ice after sonication in the extraction solution. After centrifugation, protein quantification was performed on the supernatant.

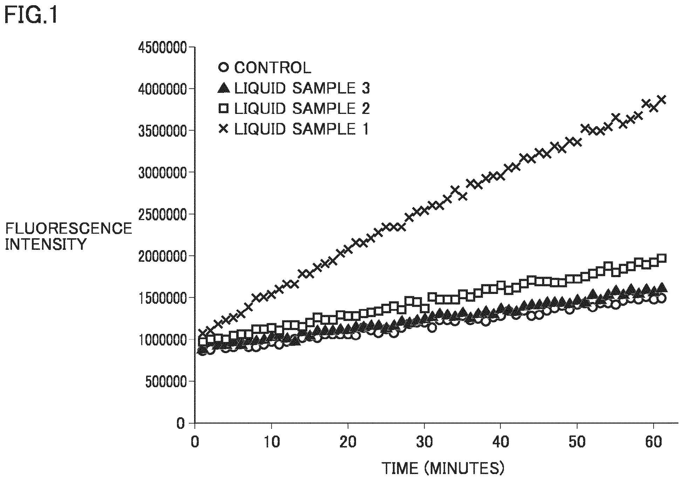

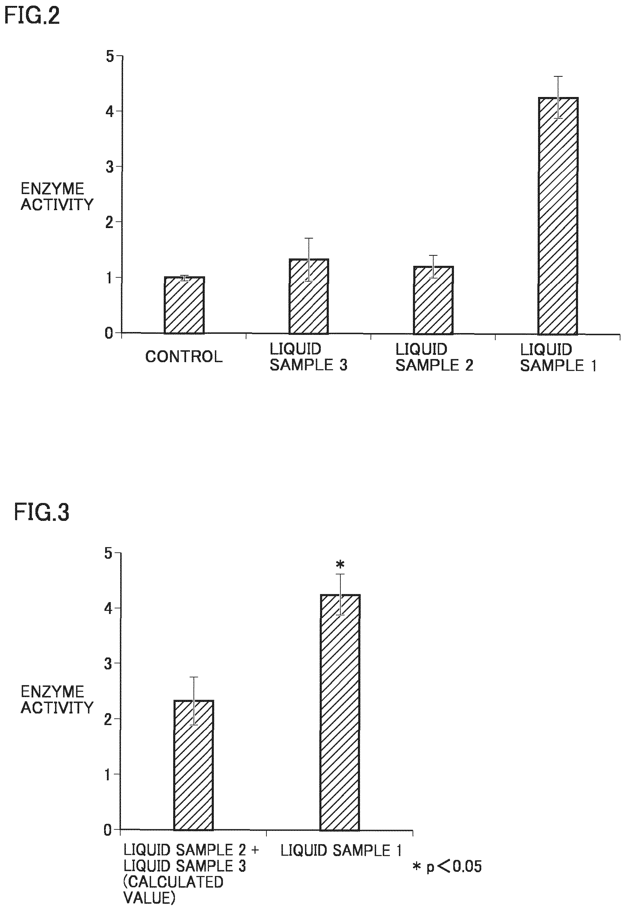

SIRT1 enzyme activity measurement was performed with SIRT1/Sir2 Deacetylase Fluorometric Assay Kit Ver. 2 (Medical & Biological Laboratories Co., Ltd.). SIRT1 enzyme having 0.02 U/mL, 25 µM NAD⁺, and 25 µM fluorescently labeled substrate were added to the supernatant adjusted to an any concentration, incubation was performed at 37°C for 60 minutes, and then fluorescence was measured at an excitation wavelength of 355 nm and an emission wavelength of 460 nm. Fig. 1 shows the results of fluorescence intensity measured at predetermined time intervals from the start of measurement. Fig. 2 shows relative values with the SIRT1 enzyme activity in the control to which no sample was added as 100 based on the fluorescence intensity after 60 minutes from the start of the measurement shown in Fig. 1. In Fig. 2, the significance probability of less than 5% (P < 0.05) is indicated by * for the significance probability P value in the t-test. Fig. 3 shows a calculated value obtained by adding the results of liquid sample 2 and liquid sample 3, and a measured value of liquid sample 1 for the result shown in Fig. 2.

From the result shown in Fig. 3, it has been found that liquid sample 1 exhibited a more pronounced SIRT1-activating action than the effect obtained by adding the SIRT1-activating actions of liquid sample 2 and liquid sample 3.

## Claims

1. A liquid composition comprising nicotinamide mononucleotide and an aronia extract.

2. The liquid composition according to claim 1, which is for suppressing aging.

3. The liquid composition according to claim 2, which has a sirtuin-activating action.

4. A composition for activating sirtuin, comprising nicotinamide mononucleotide and an aronia extract.

5. The composition according to claim 4, wherein the sirtuin is sirtuin 1.

6. The composition according to any one of claims 1 to 5, which is for oral intake.
